# EUROPEAN PATENT SPECIFICATION

(11) **EP 2 033 671 B1**
(45) Date of publication and mention of the grant of the patent: **26.01.2011**
(21) Application number: 08163474.3
(22) Date of filing: 02.09.2008
(51) Int. Cl.: A61M 1/02, A61M 1/30

(54) **Kit for oxygen-ozone therapy**
Kit für Sauerstoff-Ozon-Therapie
Kit de thérapie à l'oxygène-ozone

(30) Priority: 05.09.2007 IT MI20071727; 04.12.2007 IT MI20072274
(43) Date of publication of application: 11.03.2009
(73) Proprietor: GOBBI FRATTINI, Ditta Paolo Giuseppe, 23035 Sondalo (SO) (IT)
(72) Inventor: Valoti, Michele, 6900, Massagno (CH)
(74) Representative: Mittler, Enrico

(56) References cited:
- EP-A- 0 596 260
- DE-A1- 4 410 411

## Description

The present invention relates to a kit for oxygen-ozone therapy.

Oxygen-ozone therapy is a technique which exploits the potential of ozone combined to oxygen to stimulate and increase the protective mechanisms against the production of free radicals and toxic substances for cells by reducing them. The percentages of oxygen and ozone are: 99.99997% oxygen and 0.00003% ozone.

This method, which after years of clinical and scientific experience has been consolidated and is widespread in many countries, also in virtue of the absence of known contraindications, in a high percentage of cases allows to solve or at least improve a number of diseases, which are otherwise strongly disabling, with significant individual and social benefits. The analgesic, anti-inflammatory, anti-viral and anti-bacterial functions are mentioned by way of mere example. The diseases which may benefit therefrom range from arthrosis to vasculopathies, up to senescence, only to mention some. It is therefore a broad use therapy.

A therapeutic method implies collecting a blood sample (approximately 30 ml) from the patient, mixing with oxygen and ozone and transfusing the mixture to the patient. The methods according to the known art provide for carrying out a first injection by means of the sample collection needle, the needle is then extracted and a second injection is carried out with another needle, clearly in a distinct point, for the transfusion to the patient of the mixture of blood treated with oxygen and ozone.

Therefore, in the known art the patient undergoes two injections.

It has also been understood that it would be better for the materials forming the various components, especially those contacting the blood, not to contain plasticizers, i.e. additives which make plastic materials such as PVC flexible in order to avoid even a minimum absorption by the organism.

It has also been noted that there is no complete kit dedicated to this practice on the market and this is compensated by combining various elements, which therefore may not have the above mentioned features.

DE 4410441 A1 discloses a set for oxygen-ozone therapy.

It is therefore the object of the present invention to provide a kit for oxygen-ozone therapy allowing to perform only one injection to the patient and consisting of dedicated elements.

In accordance to the invention, this object is achieved by a kit for oxygen-ozone therapy including a blood collection and mixing bag with an inlet for the blood to be treated and at least one first normally closed opening provided with a first clamp and with a connector for introducing a mixture of oxygen-ozone, a small tube, connected to said inlet, to convey the collected blood to the bag, the small tube including a Y-shaped joint provided with a side inlet point, a second clamp placed on the small tube between the inlet and the Y-shaped joint, said kit including a transfuser which may be connected to said at least one first opening, by means of said connector, and to the Y-shaped joint by means of said side inlet point, after the introduction of said oxygen-ozone mixture to serve, once said first clamp has been opened and said second clamp has been closed as a bypass element with respect to the small tube and to allow a transfusion of oxygen-ozone treated blood to a patient by means of the same needle used for the collection of blood without removing it from the patient

These and other features of the present invention will become more apparent from the following detailed description of two embodiments thereof, shown by no way of limitation in the accompanying drawings, in which:
Figure 1 shows a front view of a first embodiment of the kit according to the invention;
Figure 2 shows a diagrammatic view of the collecting step;
Figure 3 shows a diagrammatic view of an anticoagulant injection;
Figure 4 shows a diagrammatic view of the mixing step of the collected blood with oxygen-ozone;
Figure 5 shows a diagrammatic view of the step of removing the mixing syringe;
Figure 6 shows a diagrammatic view of the step of transfusing the blood mixed with oxygen-ozone;
Figure 7 shows a front view of a second embodiment of the kit according to the invention;
Figure 8 shows a front view of the kit of Fig. 7 during a step of opening the communication between the collection needle and the small tube which connects it to the bag;
Figure 9 shows a front view of the kit of Fig. 7 during a step of collecting blood;
Figure 10 shows a front view of the kit of Fig. 7 during a step of injecting anti-coagulant;
Figure 11 shows a front view of the kit of Fig. 7 during a step of mixing oxygen-ozone with the collected blood;
Figure 12 shows a front view of the kit of Fig. 7 during a transfusion of the blood and oxygen-ozone mixture.

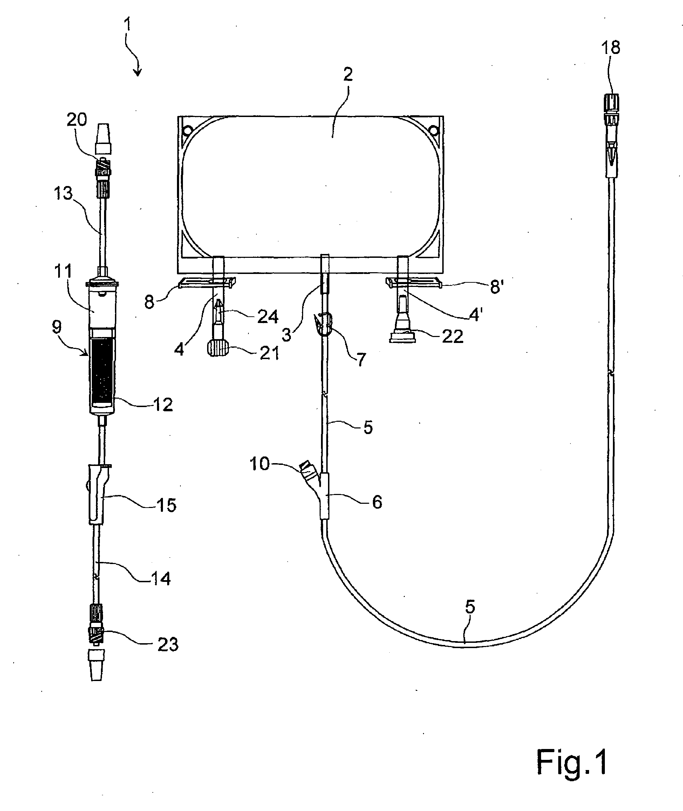

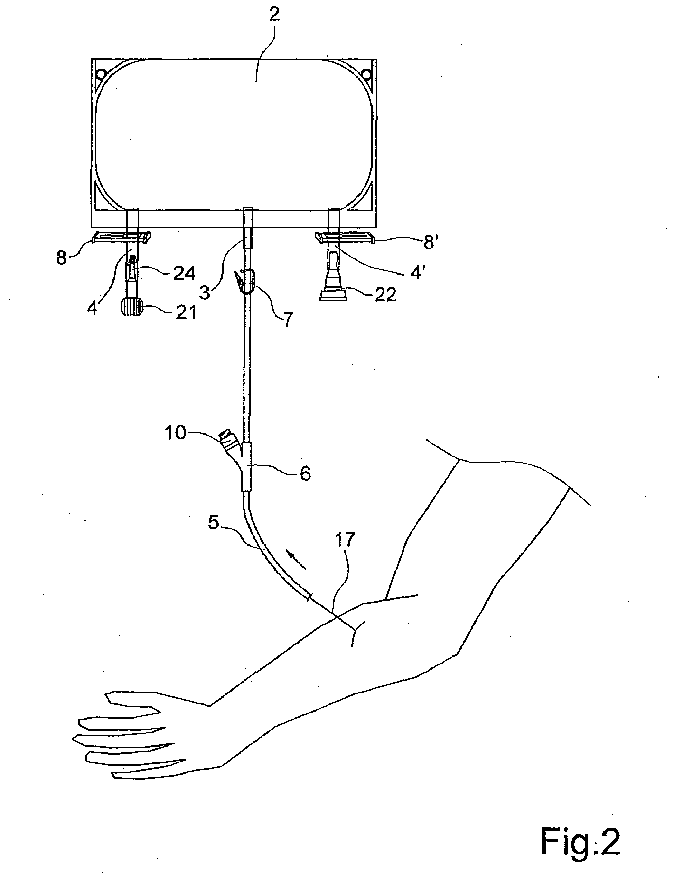

With reference to Figures 1 and 2, a kit 1 for oxygen-ozone therapy may be noted including a bag 2 for the collection of blood with an inlet 3 for the blood to be treated and a first opening 4 provided with a screw connector 21 and a first closing clamp 8 for the inlet of the treatment fluid. The inlet 3 is provided with a second closing clamp 7. The bag 2 displays an accessory opening 4', provided with a connector 22 of the "vial-stopper" type, i.e. a stopper provided with a rubber cap that may be perforated by the syringe needle, for the introduction of medicaments. The accessory opening 4' is also provided with a third closing clamp 8'.

The first opening 4 may be provided with a hermetic inner element 24 that may be broken manually when used and may easily be recognised in the known art, for instance in IT 1339433.

Normally, the openings 4 and 4' are closed by respective clamps 8 and 8'. The accessory opening 4' may be used both as an alternative to the first opening 4, for emptying the bag 2, in case the first connection is not successful, and for the introduction of possible medicaments into the bag 2.

A small tube 5 is connected to the inlet 3 and to a connector 18 which may be connected to a needle 17 to convey the blood collected by means of the needle 17 to the bag 2, and the small tube 5 is interrupted by a Y-shaped joint 6 provided with a side inlet point 10 of the screw type, which allows the introduction of fluids without perforation needles; the clamp 7 may be adjusted in an open or closed position to interrupt or allow the blood passing towards the bag 2, and is placed between the Y-shaped joint 6 and the inlet 3. Furthermore, a transfuser 9 may be noted including a transfusion vessel 11, a filter 12, a small inlet tube 13, a small outlet tube 14 provided with a flow adjuster 15, advantageously of the roller type with a slide. Advantageously, the small tubes 13 and 14 are provided with further connectors 20 and 23 for the connection to the opening 4 (or 4') and to the inlet point 10, respectively.

With reference to Figure 2, the first step of collecting blood from the patient is noted: both clamps 8 and 8' are closed and the clamp 7 is open, the collection is performed by means of the needle 17, the small tube 5, the Y-shaped joint 6 and the further portion of small tube 5, whereby the blood pressure fills the bag 2. The transfuser 9 is still not connected.

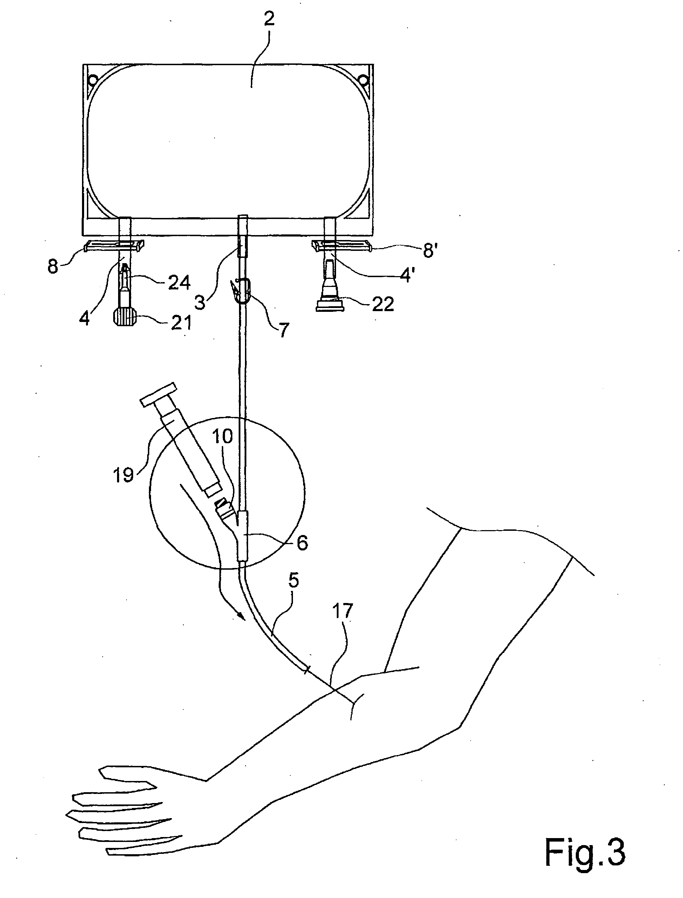

With reference now to Figure 3, the final step of the blood collection is considered. The clamp 7 is also closed, thus interrupting the flow of blood to the bag 2. An anticoagulant, such as heparin, is administered by means of a syringe 19 inserted into the inlet point 10 of the Y-shaped joint 6.

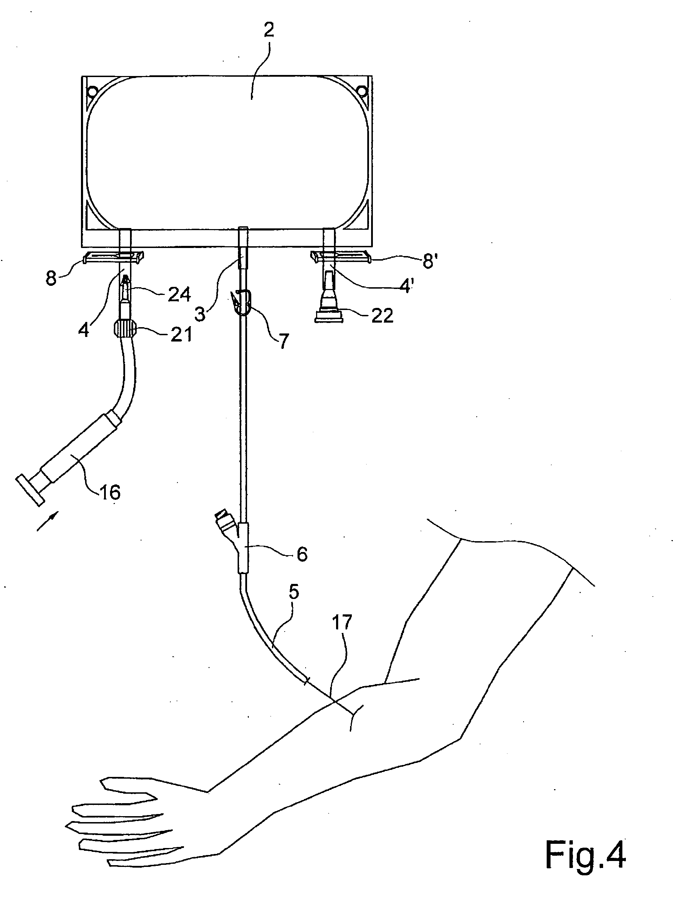

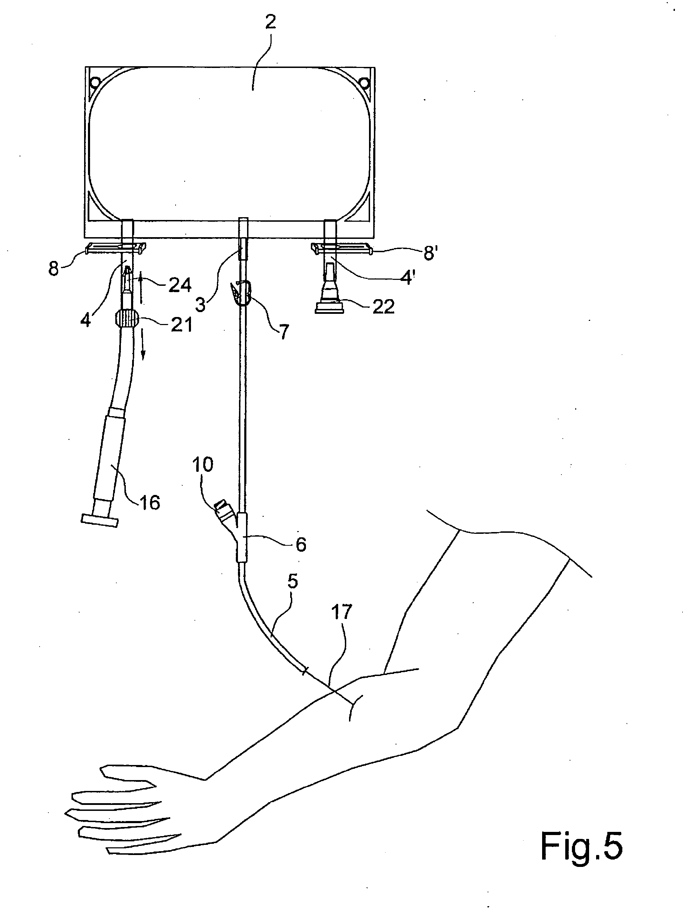

With reference now to Figures 4 and 5, the step of mixing the patient's blood with oxygen-ozone may be noted. The clamp 7 being always closed, the oxygen-ozone mixture is injected into the bag 2 by means of a syringe 16, from the opening 4 with the clamp 8 or, as an alternative, from the opening 4' with the clamp 8' open. At the end of the injection the clamp 8 or 8' is also closed and the syringe 16 is removed.

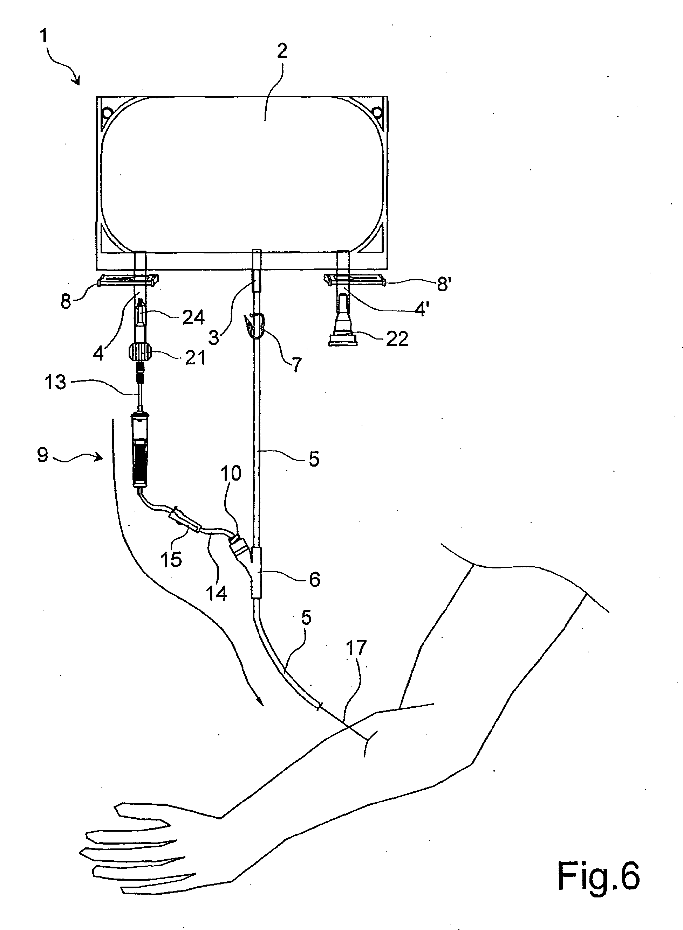

Figure 6 depicts the last treating step which implies the connection of the small inlet tube 13 of the transfuser 9 to the opening 4 (or 4') by means of the connector 20, and the small outlet tube 14 to the inlet point 10 by means of the connector 23. At this point, the clamp 8 (or 8') corresponding to the opening 4 (or 4'), to which the transfuser 9 is connected, is opened and when the clamp 7 is closed the treated blood flows out in the transfusion vessel 11, therefrom to the filter 12 and then to the patient. Once the transfuser 9 is connected to the bag 2, care should be taken to let it completely fill with blood, including the vessel 11 and the small tube 14, before connecting it by means of the connector 23 connected to the inlet point 10 , to the small tube 5 and then to the patient, in order to avoid the introduction of air bubbles into the blood circulation of the patient.

It should be noted that the needle 17 has not been removed during the whole operation.

The bag 2 displays the advantageous feature of developing horizontally, i.e. having a length longer than its height, in order to facilitate the mixing of the oxygen-ozone.

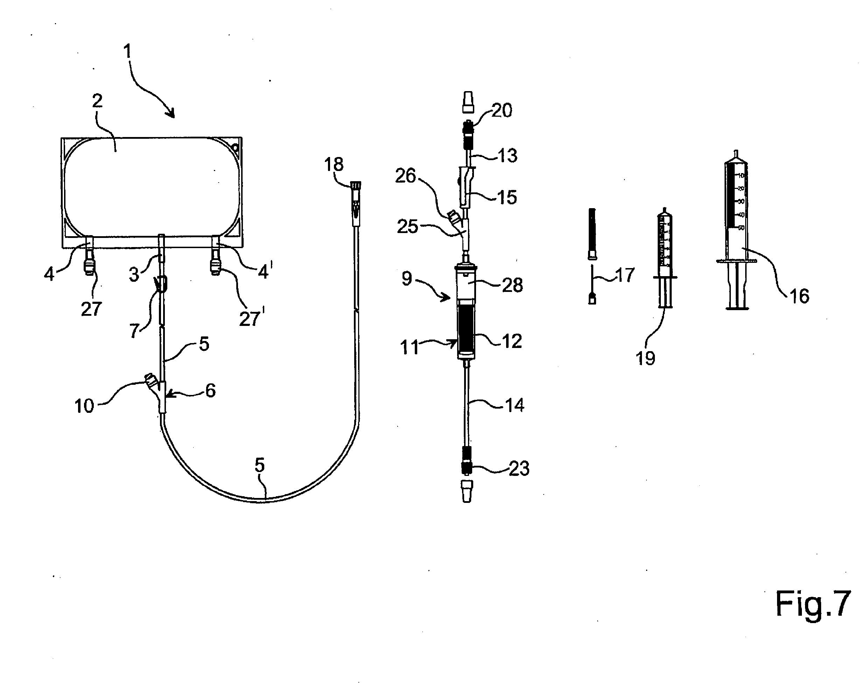

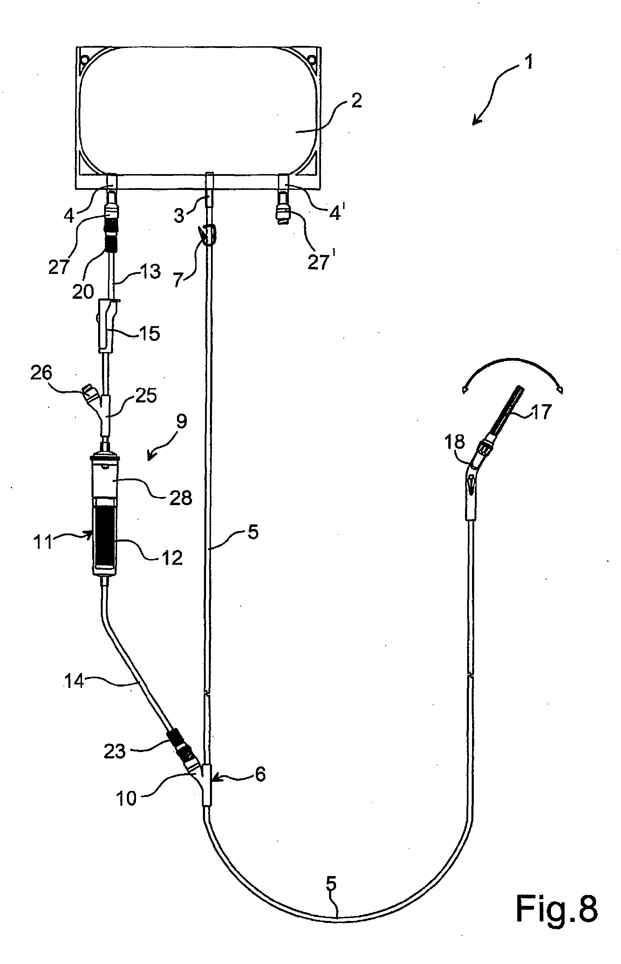

With reference to Figures 7 and 8, in which the same reference numbers have been used to designate similar parts, a kit 1 for oxygen-ozone therapy is shown including a bag 2 for the collection of blood with an inlet 3 and a first opening 4', provided with a first connector 27' which may be opened and closed, The bag 2 displays a second opening 4, provided with a second connector 27 which may also be opened or closed.

The connectors 27 and 27' are preferably of the screw-type with an automatic opening and closing, which allows the introduction of fluids and gas without the use of perforation needles.

A small tube 5 provided with a closing clamp 7 is connected to the inlet 3 and to a connector 18 for the needle, also provided with an element which may be broken manually when used and connected to a needle 17, to convey the blood collected by means of the needle 17 to the bag 2, and the small tube 5 is interrupted by a Y-shaped joint 6 provided with a side inlet point 10 of the screw type that allows the introduction of fluids without perforation needles. The clamp 7 may be shifted in the closing or opening position to interrupt or allow the blood passing towards the bag 2, and is placed between the Y-shaped joint 6 and the inlet 3.

A transfuser 9 with a vessel 11 provided with an anti-bubble 28 to avoid air bubbles in the patient's circulation, a filter 12, a small outlet tube 14, a small inlet tube 13 provided with a flow adjuster 15, advantageously of the roller type with a slide, and a second Y-shaped joint 25 with a side inlet point 26 placed between the flow adjuster 15 and the transfuser 9 may also be noted. The small tubes 13 and 14 are provided with further connectors 20 and 23 for the connection to the opening 4 (or 4' as an optional choice in case of failure of the opening 4) and to the inlet point 10 of the Y-shaped joint 6, respectively.

Further components of the kit are an anticoagulant syringe 19 and an oxygen-ozone syringe 16.

Figure 8 shows the step of breaking the seal of the needle 17 by means of a manual bending of the connector 18 for the needle which, by breaking the inner hermetic element, allows the communication between the needle 17 and the rest of the circuit to be opened, the kit 1 being mounted with the transfuser 9 between the connector 27 and the side inlet point 10 of the Y-shaped joint 6.

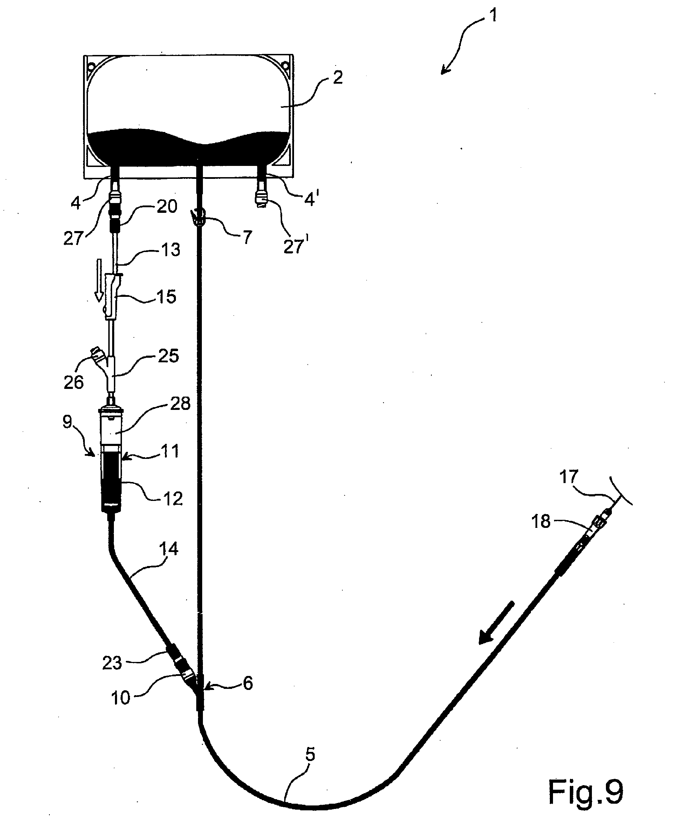

With reference to Figure 9, in relation to the treatment method, again with the transfuser 9 connected by means of the second connector 27 to the bag 2 and to the side inlet point 10 of the Y-shaped joint 6, with an open flow adjuster 15 and an open clamp 7, the insertion of the needle 17 into the arm of the patient may be noted, with the subsequent flow of blood to the bag 2. The flow adjuster 15 remains open until the preset level of blood is achieved in the vessel 12 of the transfuser 9, after which it is closed.

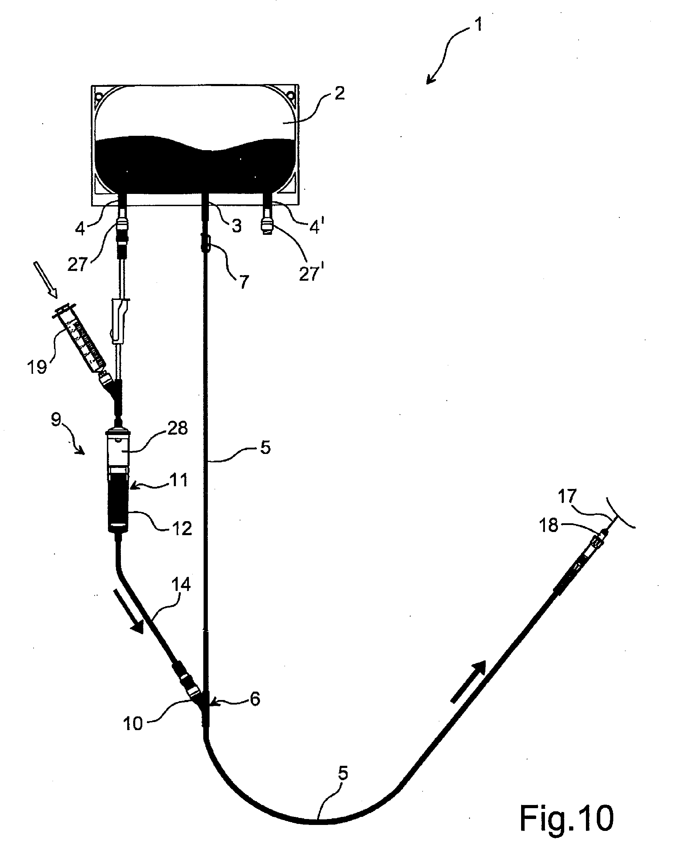

Subsequently, with reference to Figure 10, the clamp 7 is closed and an anticoagulant is injected by means of the syringe 19 introduced into the side inlet point 26 of the Y-shaped joint 25.

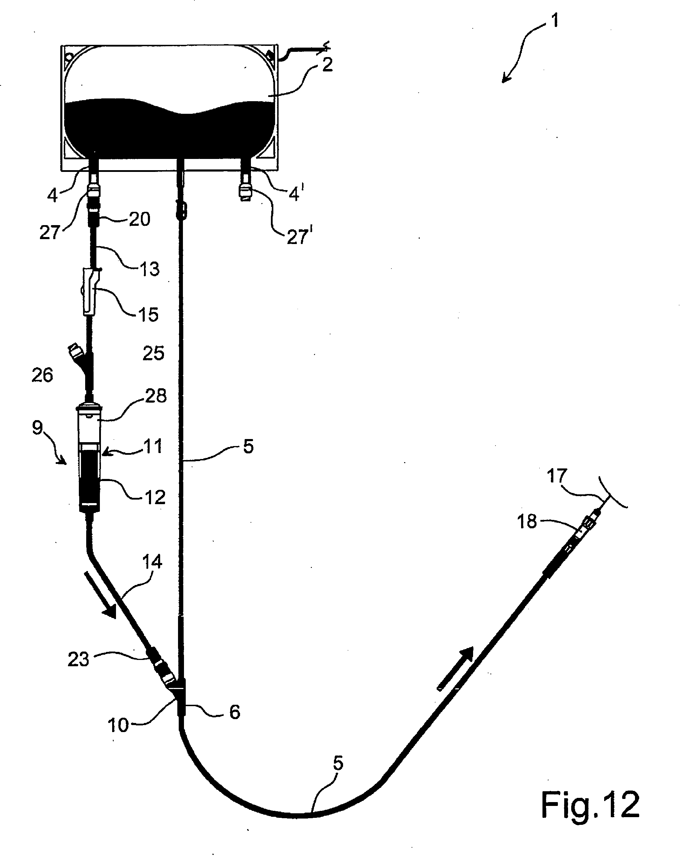

The first opening 4' is then connected to the syringe 16 with the oxygen-ozone mixture to be mixed and the content of the syringe is injected into the bag 2 by means of the connector 27', to mix oxygen-ozone with the collected blood. The syringe 16 is then removed from the opening 4' while closing the connector 27' and by means of the connector 27 and the flow adjuster 15, which is opened again, the blood of the bag is let flow to the transfuser 9 to perform the transfusion of the blood treated with the oxygen-ozone mixture through the needle 17, which remains inserted into the patient (Figure 12).

It should be noted that the first opening 4' may be used as an alternative to the second opening 4 for connecting the transfuser branch 9 to the bag 2.

The bag 2 displays the advantageous feature of developing horizontally, i.e. having a length longer than its height, in order to facilitate the mixing of the oxygen-ozone. Advantageously, the flow adjuster 15 is of the roller type with a slide.

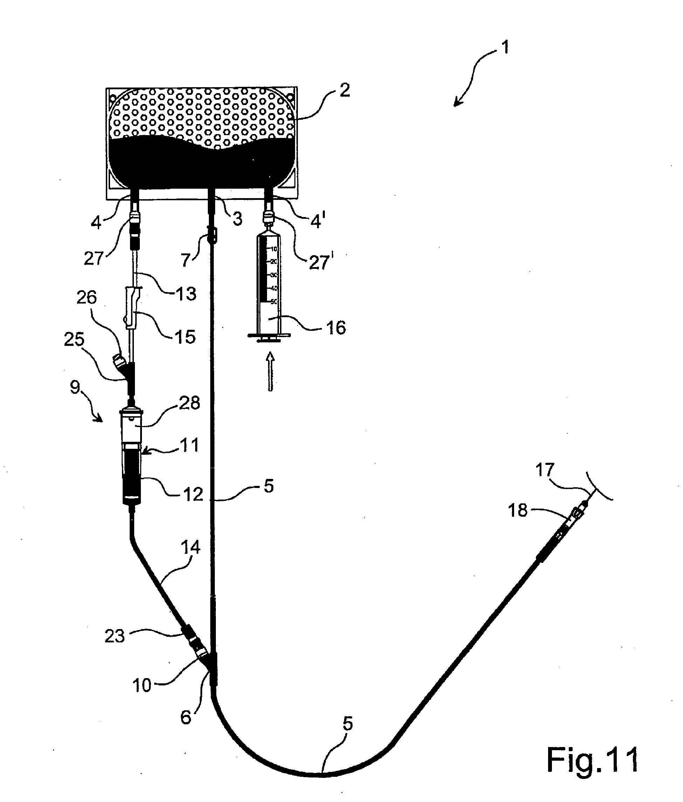

It should also be noted that the kit of Figures 7-12 forms the first self-transfusion kit for a closed circuit oxygen-ozone therapy, i.e. with a collection branch (small tube 5) and the transfuser branch (with the transfuser 9) always connected to the bag 2 and to each other. This takes place in virtue of the presence of a flow adjuster 15 on the branch with the transfuser 9 between the transfuser 9 and the bag 2, which flow adjuster is capable of interrupting the flow in the collecting step, and in virtue of the presence of the second Y-shaped joint 25. This innovative and advantageous feature allows a higher sterility as the transfuser branch is not removed and reconnected.

Finally, it should be noted that the kit according to the invention, in virtue of the fact that the patient's blood is filling the bag 2 and, partially, the transfuser 9 before the treatment and in virtue of the Y-shaped joint 25 placed above the anti-bubble 28 and the vessel 12, avoids the formation of gaseous emboli which would otherwise be possible in the case the fluid of the bag is directly introduced into the patient's vein.

## Claims

1. A kit (1) for oxygen-ozone therapy including a blood collection and mixing bag (2) with an inlet (3) for the blood to be treated and at least one first normally closed opening (4, 4') provided with a first clamp (8, 8') and with a connector (21, 22; 27, 27') for introducing a mixture of oxygen-ozone, a small tube (5) connected to said inlet (3) to convey the collected blood to the bag (2), the small tube (5) including a Y-shaped joint (6) provided with a side inlet point (10), a second clamp (7) placed on the tube (5) between the inlet (3) and the Y-shaped joint (6), said kit including a transfuser (9) which may be connected to said at least one first opening (4, 4'), by means of said connector (21, 22), and to the Y-shaped joint (6) by means of said side inlet point (10), after the introduction of said oxygen-ozone mixture to serve, once said first clamp (8, 8') has been opened and said second clamp (7) has been closed as a bypass element with respect to the small tube (5) and to allow a transfusion of oxygen-ozone treated blood to the patient by means of the same needle used for the collection without removing it from the patient.

2. A kit (1) according to claim 1, **characterised in that** the bag (2) includes a second opening (4') with a third closing clamp (8').

3. A kit (1) according to claim 1 or 2, **characterised in that** the transfuser (9) includes a transfusion vessel (11), a filter (12), a small inlet tube (13), a small outlet tube (14) and a flow adjuster (15) thereon.

4. A kit (1) according to claim 3, **characterised in that** the flow adjuster (15) is of the roller type with a slide.

5. A kit (1) according to any one of the preceding claims, **characterised in that** it includes a syringe (16) to inject the oxygen-ozone mixture into the bag (2) by means of said at least one opening (4, 4').

6. A kit (1) according to any one of the preceding claims, **characterised in that** it includes a needle (17) and a connector (18) to be connected to the small tube (5) for the collection of blood and the transfusion.

7. A kit (1) according to any one of the preceding claims, **characterised in that** the opening (4, 4') is provided with a hermetic inner element (24) which may be broken when used.

8. A kit (1) according to any one of the claims from 2 to 7, **characterised in that** the opening (4, 4') is provided with a vial-stop type connector.

9. A kit (1) according to any one of the claims from 2 to 8, **characterised in that** the second opening (4, 4') is used as an alternative to the first opening (4, 4') for emptying the bag (2) in case the emptying through the first opening (4, 4') is not successful, thus opening the second clamp (8').

10. A kit (1) according to any one of the claims from 1 to 8, **characterised in that** the opening (4, 4') is used for the introduction of medicaments into the bag.

11. A kit (1) according to any one of the preceding claims, **characterised in that** it includes two further connectors (20, 21; 23) at the ends of the small inlet tube (13) and of the small outlet tube (14).

12. A kit (1) according to any one of the preceding claims, **characterised in that** it is made of phthalate-free materials.

13. A kit (1) according to any one of the preceding claims, **characterised in that** the bag (2) develops horizontally in order to facilitate the mixing of oxygen-ozone.

14. A kit (1) according to claim 1, **characterized in that** said transfuser (9) is provided with a transfusion vessel (11) which may be connected by means of first (20) and second (23) connectors to one of said openings (4, 4') and to said inlet point (10) of said Y-shaped joint (6), said transfuser (9) further including a Y-shaped joint (25) inserted between said transfusion vessel (11) and said first connector (20) of the transfuser (9) and provided with an inlet point (26) for an anticoagulant substance and a flow adjuster (15) placed between said first connector (20) and said second Y-shaped joint (25) of the transfuser (9).

15. A kit (1) according to any one of the preceding claims, **characterised in that** the transfuser (9) is provided with an anti-bubble (28).

## Patentansprüche

1. Set (1) für eine Sauerstoff-Ozon-Therapie, das einen Blutsammel- und Mischbeutel (2) mit einem Einlass (3) für das zu behandelnde Blut und mindestens einer ersten normalgeschlossenen Öffnung (4, 41), die mit einer ersten Klammer (8, 8') und mit einem Stecker (21, 22; 27, 27') zum Einführen eines Gemischs aus Sauerstoff-Ozon versehen ist, eine kleine Röhre (5), die mit dem Einlass (3) verbunden ist, um das gesammelte Blut zu dem Beutel (2) zuzuführen, wobei die kleine Röhre (5) ein Y-förmiges Anschlussstück (6), das mit einem Seiteneinlasspunkt (10) und einer zweiten Klammer (7) versehen ist, die an der Röhre (5) zwischen dem Einlass (3) und dem Y-förmigen Anschlussstück (6) platziert ist, wobei das Kit einen Transfusor (9) aufweist, der mit der zumindest einen ersten Öffnung (4, 4') durch den Stecker (21, 22) und mit dem Y-förmigen Anschlussstück (6) mittels des Seiteneinlasspunktes (10) nach der Einführung des Sauerstoff-Ozon-Gemischs verbunden werden kann, um, nachdem die erste Klammer (8, 8') geöffnet wurde und die zweite Klammer (7) geschlossen wurde, als ein Bypass-Element hinsichtlich der kleinen Röhre (5) zu dienen und um eine Transfusion des mit Sauerstoff-Ozon behandelten Bluts zu dem Patienten mittels derselben Nadel zu ermöglichen, die für das Sammeln verwendet wurde, ohne dass diese aus dem Patienten entfernt wird.

2. Set (1) gemäß Anspruch 1, **dadurch gekennzeichnet, dass** der Beutel (2) eine zweite Öffnung (4') mit einer dritten Schließklammer (8') aufweist.

3. Set (1) gemäß Anspruch 1 oder 2, **dadurch gekennzeichnet, dass** der Transfusor (9) ein Transfusionsgefäß (11), einen Filter (12), eine kleine Einlassröhre (13), eine kleine Auslassröhre (14) und einen Strömungseinsteller (15) daran aufweist.

4. Set (1) gemäß Anspruch 3, **dadurch gekennzeichnet, dass** der Strömungseinsteller (15) in einer Walzenbauart mit einem Gleiter ausgebildet ist.

5. Set (1) gemäß einem der vorherigen Ansprüche, **dadurch gekennzeichnet, dass** es eine Spritze (16) aufweist, um das Sauerstoff-Ozon-Gemisch in den Beutel (2) mittels der zumindest einen Öffnung (4, 4') einzuspritzen.

6. Set (1) gemäß einem der vorherigen Ansprüche, **dadurch gekennzeichnet, dass** es eine Nadel (17) und einen Stecker (18) aufweist, der mit der kleinen Röhre (5) zum Sammeln des Bluts und der Transfusion zu verbinden ist.

7. Set (1) gemäß einem der vorherigen Ansprüche, **dadurch gekennzeichnet, dass** die Öffnung (4, 4') mit einem hermetischen Innenelement (24) versehen ist, das beim Gebrauch gebrochen werden kann.

8. Set (1) gemäß einem der Ansprüche 2 bis 7, **dadurch gekennzeichnet, dass** die Öffnung (4, 4') mit einem Stecker der Ampullenstopp-Bauart versehen ist.

9. Set (1) gemäß einem der Ansprüche 2 bis 8, **dadurch gekennzeichnet, dass** die zweite Öffnung (4, 4') als eine Alternative zu der ersten Öffnung (4, 4') zum Entleeren des Beutels (2) verwendet wird, falls das Entleeren durch die erste Öffnung (4, 4') nicht erfolgreich ist, so dass die zweite Klammer (8') geöffnet wird.

10. Set (1) gemäß einem der Ansprüche 1 bis 8, **dadurch gekennzeichnet, dass** die Öffnung (4, 4') für die Einführung von Medikamenten in den Beutel verwendet wird.

11. Set (1) gemäß einem der vorherigen Ansprüche, **dadurch gekennzeichnet, dass** es zwei weitere Stecker (20, 21; 23) an den Enden der kleinen Einlassröhre (13) und der kleinen Auslassröhre (14) aufweist.

12. Set (1) gemäß einem der vorherigen Ansprüche, **dadurch gekennzeichnet, dass** es aus Phthalat-freien Materialien besteht.

13. Set (1) gemäß einem der vorherigen Ansprüche, **dadurch gekennzeichnet, dass** sich der Beutel (2) horizontal abwickelt, um das Mischen Sauerstoff-Ozons zu erleichtern.

14. Set (1) gemäß Anspruch 1, **dadurch gekennzeichnet, dass** der Transfusor (9) mit einem Transfusionsgefäß (11) versehen ist, das mittels eines ersten (20) und eines zweiten (23) Steckers mit einer der Öffnungen (4, 4') und dem Einlasspunkt (10) des Y-förmigen Anschlussstücks (6) verbunden werden kann, wobei der Transfusor (9) des Weiteren ein Y-förmiges Anschlussstück (25) aufweist, das zwischen dem Transfusionsgefäß (11) und dem ersten Stecker (20) des Transfusors (9) eingefügt ist und mit einem Einlasspunkt (26) für eine Antigerinnungssubstanz und einem Strömungseinsteller (15) versehen ist, der zwischen dem ersten Stecker (20) und dem zweiten Y-förmigen Anschlussstück (25) des Transfusors (9) platziert ist.

15. Set (1) gemäß einem der vorherigen Ansprüche, **dadurch gekennzeichnet, dass** der Transfusor (9) mit einer Antiblasenbildungsvorrichtung (28) versehen ist.

## Revendications

1. Kit (1) pour une thérapie à l'oxygène-ozone comprenant un sachet de collecte et de mélange de sang (2) avec une entrée (3) pour le sang à traiter et au moins une première ouverture normalement fermée (4, 4') prévue avec une première pince (8, 8') et avec un connecteur (21, 22 ; 27, 27') pour introduire un mélange d'oxygène-ozone, un petit tube (5) raccordé à ladite entrée (3) pour transporter le sang collecté jusqu'au sachet (2), le petit tube (5) comprenant un joint en forme de Y (6) prévu avec un point d'entrée latéral (10), une deuxième pince (7) placée sur le tube (5) entre l'entrée (3) et le joint en forme de Y (6), ledit kit comprenant un dispositif de transfusion (9) qui peut être raccordé à ladite au moins une première ouverture (4, 4') au moyen dudit connecteur (21, 22) et au joint en forme de Y (6) au moyen dudit point d'entrée latéral (10), après l'introduction dudit mélange d'oxygène-ozone pour servir, une fois que ladite première pince (8, 8') a été ouverte et que ladite deuxième pince (7) a été fermée, d'élément de dérivation par rapport au petit tube (5) et pour permettre une transfusion de sang traité à l'oxygène-ozone au patient, au moyen de la même aiguille utilisée pour la collecte sans la retirer du patient.

2. Kit (1) selon la revendication 1, **caractérisé en ce que** le sachet (2) comprend une deuxième ouverture (4') avec une troisième pince de fermeture (8').

3. Kit (1) selon la revendication 1 ou 2, **caractérisé en ce que** le dispositif de transfusion (9) comprend un récipient de transfusion (11), un filtre (12), 1 un petit tube d'entrée (13), un petit tube de sortie (14) et un ajusteur de débit (15) sur ce dernier.

4. Kit (1) selon la revendication 3, **caractérisé en ce que** l'ajusteur de débit (15) est du type à molette avec un curseur.

5. Kit (1) selon l'une quelconque des revendications précédentes, **caractérisé en ce qu'**il comprend une seringue (16) pour injecter le mélange d'oxygène-ozone dans le sachet (2) au moyen de ladite au moins une ouverture (4, 4').

6. Kit (1) selon l'une quelconque des revendications précédentes, **caractérisé en ce qu'**il comprend une aiguille (17) et un connecteur (18) à raccorder au petit tube (5) pour la collecte du sang et la transfusion.

7. Kit (1) selon l'une quelconque des revendications précédentes, **caractérisé en ce que** l'ouverture (4, 4') est prévue avec un élément interne hermétique (24) qui peut être cassé lorsqu'il est utilisé.

8. Kit (1) selon l'une quelconque des revendications 2 à 7, **caractérisé en ce que** l'ouverture (4, 4') est prévue avec un connecteur de type à bouchon.

9. Kit (1) selon l'une quelconque des revendications 2 à 8, **caractérisé en ce que** la deuxième ouverture (4, 4') est utilisée à titre de variante à la première ouverture (4, 4') pour vider le sachet (2) dans le cas dans lequel le vidage par la première ouverture (4, 4') est infructueux, ouvrant ainsi la deuxième pince (8').

10. Kit (1) selon l'une quelconque des revendications 1 à 8, **caractérisé en ce que** l'ouverture (4, 4') est utilisée pour l'introduction des médicaments dans le sachet.

11. Kit (1) selon l'une quelconque des revendications précédentes, **caractérisé en ce qu'**il comprend en outre deux connecteurs supplémentaires (20, 21 ; 23) aux extrémités du petit tube d'entrée (13) et du petit tube de sortie (14).

12. Kit (1) selon l'une quelconque des revendications précédentes, **caractérisé en ce qu'**il est réalisé à partir de matériaux dépourvus de phtalate.

13. Kit (1) selon l'une quelconque des revendications précédentes, **caractérisé en ce que** le sachet (2) se développe horizontalement afin de faciliter le mélange de l'oxygène-ozone.

14. Kit (1) selon la revendication 1, **caractérisé en ce que** ledit dispositif de transfusion (9) est prévu avec un récipient de transfusion (11) qui peut être raccordé au moyen des premier (20) et deuxième (23) connecteurs à l'une desdites ouvertures (4, 4') et audit point d'entrée (10) dudit joint en forme de Y (6), ledit dispositif de transfusion (9) comprenant en outre un joint en forme de Y (25) inséré entre ledit récipient de transfusion (11) et ledit premier connecteur (20) du dispositif de transfusion (9) et prévu avec un point d'entrée (26) pour une substance anticoagulante et un ajusteur de débit (15) placé entre ledit premier connecteur (20) et ledit deuxième joint en forme de Y (25) du dispositif de transfusion (9),

15. Kit (1) selon l'une quelconque des revendications précédentes, **caractérisé en ce que** le dispositif de transfusion (9) est prévu avec un dispositif anti-bulle (28).
